# EUROPÄISCHE PATENTANMELDUNG

(11) **EP 4 406 511 A1**
(43) Veröffentlichungstag der Anmeldung: **31.07.2024**
(21) Anmeldenummer: 23153781.2
(22) Anmeldetag: 27.01.2023
(51) Int. Cl.: A61F 2/20

(54) **KEHLKOPFPROTHESE**

(71) Anmelder: Proth-O-Type GmbH, 46242 Bottrop (DE)
(72) Erfinder: Helgers, Klaus Uwe, 44139 Dortmund (DE); Nazaradeh, Denis, 46236 Bottrop (DE); Nazaradeh, Fridun, 46242 Bottrop (DE); Eckermann, Claus, 46284 Dorsten (DE)
(74) Vertreter: Seyer & Nobbe Patentanwälte PartmbB

(57) **Zusammenfassung**

Die vorliegende Erfindung betrifft eine Kehlkopfprothese zur Implantation in die Kavität eines Patienten nach der Entfernung des Kehlkopfes.

## Beschreibung

Die vorliegende Erfindung betrifft eine Kehlkopfprothese mit den Merkmalen des Hauptanspruches.

Im Stand der Technik sind Kehlkopfprothesen und Elemente einer Kehlkopfprothese für Patienten nach einer Kehlkopfentfernung bereits bekannt. So beschreibt das Gebrauchsmuster DE 20 2004 010 382 U1 eine Vorrichtung zur Nachbildung der natürlichen Atemwegsfunktionen. In der DE 20 2004 010 360 U1 wird eine Kehldeckelknorpelnachbildung vorgeschlagen. DE 20 2004 010 342 U1 offenbart eine Kehlkopfprothese mit einer Vorrichtung zur Reproduktion natürlicher Stimmen, und DE 20 2004 010 341 U1 beschreibt eine Vorrichtung zur digitalen Konservierung und Verwaltung individueller stimmhafter Anteile der menschlichen Stimme.

Eine Weiterentwicklung einer Kehlkopfprothese wird vorgeschlagen in der DE 20 2007 006 203 U1, die ein im Körper stationiertes Modul als Stationsmodul und ein Mobilmodul umfasst, wobei das Mobilmodul mit einer Steck-Drehverbindung in das Stationsmodul eingesetzt wird.

Den im Stand der Technik vorgeschlagenen Lösungen ist jedoch gemein, dass diese eine für den Gebrauch nicht zufriedenstellende Atemunterstützungsfunktion für den Patienten mit sich bringen. Es besteht daher ein Bedarf an einer Kehlkopfprothese, die einen dauerhaften und für den Benutzer angenehmeren Gebrauch im täglichen Leben erlaubt.

Diese Aufgabe der Erfindung wird gelöst durch Bereitstellung einer Kehlkopfprothese mit den Merkmalen des Hauptanspruches. So betrifft die Erfindung eine Kehlkopfprothese mit einem im Wesentlichen hohlzylindrischen Prothesenhohlkörper mit einer durchgehenden Ausnehmung, wobei in der Ausnehmung in dem der Luftröhre in der Einbauposition zugewandten Abschnitt (unterer Abschnitt) der Ausnehmung zumindest eine Ventilklappe angeordnet ist, deren Öffnungswinkel und/oder der auf die Ventilklappe bei Auslenkung der Ventilklappe aus einer Ausgangsposition wirkenden Rückstellkraft, vorzugsweise elektromotorisch, einstellbar ist,
wobei in dem der Luftröhre in der Einbauposition abgewandten Abschnitt (oberer Abschnitt) der Ausnehmung zumindest eine Ventilklappe angeordnet ist, die unter Beaufschlagung mit Luftdruck gegen eine Rückstellkraft, die vorzugsweise elektromotorisch einstellbar sein kann, zwischen zumindest einer offenen Position und einer geschlossenen Position beweglich ist,
am unteren Ende der Ausnehmung unterhalb der mindestens einen Ventilklappe ein Luftstromsensor angeordnet ist,
in der Ausnehmung zwischen der unteren mindestens einen Ventilklappe und der oberen mindestens einen Ventilklappe optional ein weiterer Luftstromsensor angeordnet ist,
wobei am oberen Ende des Prothesenhohlkörpers ein künstlicher Kehldeckel, der zum Verschluss der Ausnehmung ausgelegt ist und der zwischen einer offenen Position und einer geschlossenen Position beweglich ist, und eine Feder, die zur Öffnung des Kehldeckels ausgelegt ist, angeordnet sind; und
wobei der Prothesenhohlkörper eine in der Regel elektronische Steuereinheit zur Einstellung der Positionen und der Rückstellkraft/Federkraft der Ventilklappen und zur elektrischen Ansteuerung der Bauelemente der Kehlkopfprothese, mindestens einen Akkumulator zur Stromversorgung, eine Transpondereinheit zur Datenübermittlung und zur Aufladung des Akkumulators sowie Schnittstellen zur Verbindung mit Nerven und/oder Muskeln des Muskel- und Sprechapparates aufweist.

Bei den im Stand bekannten Lösungen hatte der Patient keinen Widerstand beim Sprechen. Die Erfindung stellt eine Kehlkopfprothese mit einem verbesserten Klappensystem bereit, das Widerstand im Luftstrom beim Sprechen erzeugt und weiterhin Funktionen wie Husten, Bauchpresse o.ä. ermöglicht.

Die Einstellung des Öffnungswinkels und/oder der auf jede Ventilklappe bei Auslenkung aus der Ausgangsposition wirkenden Rückstellkraft/Federkraft kann erfindungsgemäß elektromotorisch oder hydraulisch mittels an der Kehlkopfprothese angeordneter oder darin integrierter Motoren oder hydraulischer Elemente erfolgen, die über die Steuereinheit angesteuert werden und die die Rückstellkraft/Federkraft auf die Ventilklappe regeln können. An der mindestens einen Ventilklappe kann auch mindestens ein Federelement vorgesehen sein, das bei einer auf die Ventilklappe wirkenden Kraft, beispielsweise infolge der Luftströmung, eine Rückstellkraft auf die Ventilklappe gegen diese Kraft ausübt. Die Rückstellkraft des Federelementes kann auch in ihrer Stärke optional mittels der an der Kehlkopfprothese angeordneten oder darin integrierten Motoren oder hydraulischer Elemente eingestellt werden.

In einer Ausführung der Kehlkopfprothese sind in dem der Luftröhre in der Einbauposition abgewandten Abschnitt (oberer Abschnitt) der Ausnehmung zumindest zwei Ventilklappen angeordnet, die unter Beaufschlagung mit Luftdruck gegen eine Federkraft, die vorzugsweise für jede der mindestens zwei Ventilklappen unterschiedlich voreingestellt/einstellbar ist, zwischen einer offenen Position und einer geschlossenen Position beweglich sind.

In einer weiteren Ausführung der Kehlkopfprothese sind in dem der Luftröhre in der Einbauposition zugewandten Abschnitt (unterer Abschnitt) der Ausnehmung zumindest zwei Ventilklappen angeordnet, deren Öffnungswinkel, vorzugsweise elektromotorisch, einstellbar sind.

Bei der letztgenannten Ausführung der Kehlkopfprothese ist von Vorteil, wenn in dem der Luftröhre in der Einbauposition abgewandten Abschnitt (oberer Abschnitt) der Ausnehmung mindestens zwei Ventilklappen angeordnet sind, die unter Beaufschlagung mit Luftdruck gegen eine einstellbare Federkraft/Rückstellkraft, die elektromotorisch einstellbar sein kann, zwischen einer zueinander offenen Position über eine zueinander geschlossene Position zu einer zweiten zueinander offenen Position beweglich sind. Dabei sind die mindestens zwei Ventilklappen bei Auslenkung vorteilhaft mit einer jeweils unterschiedlichen einstellbaren Federkraft beaufschlagbar. Dabei können die zumindest zwei Ventilklappen an ihren Aufhängepunkten auch beweglich so gelagert sein, dass die jeweilige Drehachse zwischen zwei Positionen senkrecht zur Drehachse verschiebbar ist und so die Ventilklappen aneinander vorbei beweglich sind, ohne die Beweglichkeit gegenseitig zu beeinträchtigen.

Die Ausnehmung der erfindungsgemäßen im Wesentlichen hohlzylindrischen Kehlkopfprothese kann einen vieleckigen, elliptischen, runden oder nahezu runden Querschnitt haben, der vom unteren Abschnitt zum oberen Abschnitt in Abhängigkeit von den verwendeten Bauelementen wie Klappen oder deren Anzahl auch unterschiedlich ausgestaltet sein kann. Die Außengeometrie der Kehlkopfprothese ist dabei im Wesentlichen zylindrisch.

Die erfindungsgemäße Kehlkopfprothese zeichnet sich durch eine Ventilklappen-Anordnung mit mindestens einer unteren Klappe und mindestens einer oberen Klappe zur Erzeugung von Überdruck und eines Widerstands beim Sprechen aus.

Dabei bildet die untere Ventilklappe, oder kurz Klappe, einen Widerstand im Luftstrom beim Sprechen. Mittels Messung der Luftströmungsgeschwindigkeit oberhalb der unteren Klappe sowie des Luftdrucks unterhalb der unteren Klappe kann die gewünschte Sprechlautstärke ermittelt werden. Erfindungsgemäß wird somit eine Klappe bereitgestellt, die die natürlichen Mechanismen beim Sprechen nachbilden kann, indem sie variabel geschlossen oder geöffnet werden kann, so dass ein unterschiedlich großer Luftwiderstand erzeugt werden kann. Daher sind vorteilhaft eine Vorrichtung zur Messung der Luftströmungsgeschwindigkeit der Luft direkt oberhalb der unteren Klappe sowie eine Vorrichtung zur Messung des Luftdrucks unterhalb der unteren Klappe angeordnet.

Anhand von mindestens einem Sensor kann so in der erfindungsgemäßen Prothese die Geschwindigkeit der Luftströmung bestimmt werden. Dabei kann durch einen unterhalb der Klappen am unteren Ende in der Einbausituation platzierten Drucksensor der Druck ermittelt werden. Weiterhin kann beispielhaft mittels eines Staurohres als Sensorelement direkt oberhalb der künstlichen Rima glottidis, also der unteren Klappe, anhand des Staudrucks und mit Hilfe der Bernoulli-Gleichung ermittelt werden, wie hoch die Strömungsgeschwindigkeit der Luft direkt oberhalb der Rima glottidis ist. Beide gemessenen Daten können zur Einstellung der Lautstärke, mit der der Patient sprechen möchte, verwendet werden.

Als weitere Parameter können die über die Nervenschnittstellen auf dem Nervus phrenicus sowie der Nervi intercostales gemessenen Werte verwendet werden, um ein forciertes Ausatmen / Sprechen zu ermitteln. Impulse von Muskeln können ebenfalls verarbeitet werden, sofern diese Muskeln nicht vollständig entfernt wurden und noch Signale zur Prozessierung abgeben.

Die obere Ventilklappe stellt ein passives Klappenventil dar, das bei stoßartiger Ausatmung zunächst schließt, um bei dann weiter ansteigendem Druck plötzlich zu öffnen und so dem Patienten das Husten zu ermöglichen. Für den richtigen Umgang mit der erfindungsgemäßen Kehlkopfprothese wird der Patient so geschult, um das Erzeugen von Überdruck und das Husten zu üben.

Das erfindungsgemäße im Prothesenhohlkörper angeordnete System mit mindestens zwei Klappen ermöglicht es, Überdruck aufzubauen, um zum Beispiel das Husten, die Defäkation und das Heben von schweren Lasten zu ermöglichen. Zusätzlich kann durch das Klappen-System ein Widerstand beim Sprechen erzeugt werden.

Die mindestens eine untere Klappe und die mindestens eine obere Klappe sind in Ruheatmung bzw. bei stummem Patienten maximal geöffnet. Um dies zu gewährleisten, können, vorzugsweise elektromotorische Antriebe, beispielsweise elektrisch in ihrer Rückstellkraft einstellbare Federn, vorgesehen sein, die die Klappen an der Wandung des Prothesenhohlkörpers anliegen lassen. Dadurch ist gewährleistet, dass die Klappen in Ruheatmung geöffnet sind und sich nicht bereits beim kleinsten Luftstrom schließen, was sehr unangenehm für den Kehlkopfprothesenträger wäre. Die Stärke der Federn ist individuell einstellbar, da jeder Mensch anders atmet und sich die Leistungsstärke der Lunge sowie das Atemzugvolumen von Mensch zu Mensch unterscheiden. Neben den Stimmparametern kann somit auch der Federdruck der in ihrer Rückstellkraft einstellbaren Federn an die Prothese übermittelt werden.

Wenn der Prothesenträger ruckartig ausatmet, schließt sich das obere Klappenventil, indem die mindestens eine Klappe schließt, oder die bevorzugt mindestens zwei Klappen aneinanderschlagen, und so wird ein Widerstand für den Luftstrom aufgebaut, wodurch sich ein Druck in der unteren Luftröhre aufbaut, gegen die die Bauchpresse arbeitet. Somit kann abdomineller Druck z.B. bei Defäkation oder beim Heben von schweren Lasten aufgebaut werden.

Steigt der Druck weiter stark an, kann sich das obere Klappenventil gegen die auf die Klappe(n) wirkende Federkraft schlagartig öffnen. Dazu können die Klappen des oberen Klappenventils so aufgehängt sein, dass die Lager der Klappen ab einem gewissen Druck zur Seite nachgeben. Dadurch können die Klappen in Richtung des ausströmenden Luftstroms aus der Lunge nach oben aufschlagen. Dadurch kann schlagartig Luft ausströmen, und der Patient hustet. Damit die Klappen bei der Rückstellung in Normalposition sich nicht gegenseitig behindern, kann vorzugsweise an zumindest einer Klappe ein Dämpfungselement vorgesehen sein, so dass die Bewegung der Klappe verlangsamt werden kann und erst die eine Klappe, dann die andere Klappe in die Öffnungsposition bewegt wird. So kann der Patient wieder husten.

Vorteilhaft weist der Prothesenhohlkörper zusätzlich einen Schallwandler nach Art eines Lautsprechers, der über die mindestens eine Steuereinheit angesteuert wird, auf.

Das Schallsignal wird in die Trachea mit Hilfe eines Trichters eingekoppelt, der über einem Lautsprecher im oberen Abschnitt der Ausnehmung an der Prothese gesichert angeordnet ist. Der Trichter hat vorzugsweise einen flexiblen Trichterhals mit einer vorgegebenen Länge. Die Länge des Trichterhalses kann auf das System, bestehend aus Lungen, Trachea, Pharynx, Mundraum und Nasennebenhöhlen des Patienten abgestimmt werden, um so bei möglichst geringer Leistungsaufnahme des Verstärkers eine optimale Lautstärke zu erzielen.

Die optimale Länge des Trichterhalses wird mit Hilfe von Messungen vor dem operativen Eingriff am Patienten, im Rahmen der Aufzeichnung der Stimmcharakteristika im Labor, z.B. mittels Systemimpulsantwort, also Dirac-Impuls und anschließender Messung in der Mundhöhle, oder durch Simulation mit Hilfe eines 3D-Modells, bestimmt

Der Trichterhals wiederum besteht vorzugsweise aus dem gleichen Material wie die Prothese. Da der Trichterhals im Luftraum der Prothese mündet, handelt es sich ebenfalls um ein bakterizides und fungizides Material.

Der Trichterhals ist vorzugsweise zwischen unterer und oberer Klappe(n), vorzugsweise direkt oberhalb der unteren Klappe in der Ausnehmung der Prothese angeordnet.

Die Erfindung betrifft ebenso ein Kit mit einer Kehlkopfprothese und mit einer Vorrichtung zur Aufladung des Akkumulators, und vorteilhaft mit einer Vorrichtung zur Kommunikation mit der Steuereinheit.

Dabei können die Vorrichtung zur Aufladung des Akkumulators und/oder die Vorrichtung zur Kommunikation mit der Steuereinheit jeweils vorzugsweise in ein Halsband für den Patienten integriert sein.

Um einen natürlich wirkenden Widerstand beim Sprechen zu bieten, müssen die Stimmlippen nachgebildet werden. Hierzu dient das untere Klappenventil, das servoelektrisch/elektromotorisch gesteuert werden kann.

Dabei wird auch sichergestellt, dass bei einer Störung (Ausfall der Elektronik, Stromausfall) das untere Klappenventil mit Hilfe von einem oder mehreren Federelementen in die maximal geöffnete Position gestellt wird. Es ist höchste Priorität, sicherzustellen, dass die Atemwege des Patienten im Falle einer Störung der Prothese stets frei bleiben!

Möchte der Patient sprechen, wird anhand von Nervenimpulsen auf dem Nervus laryngeus recurrens (oder etwaig verbliebener Muskulatur, was nach tumorchirurgischen Eingriffen sehr unwahrscheinlich sein dürfte) mit Zusatzinformationen vom Nervus phrenicus sowie den Nervi intercostales dieser Wunsch von der Prothese erfasst. Anhand der Nervenimpulse stellt die Prothese mit Hilfe von Servomotoren die unteren Klappen so ein, dass ein geringer Widerstand für den Luftstrom erzeugt wird. So kann der Patient gegen einen Widerstand "ansprechen". Diesen Widerstand haben vor dem Eingriff die Stimmlippen dargestellt.

Wenn Muskeln noch vorhanden sind, können Impulse des Muskels Musculus cricoarytaenoideus posterior alternativ hinzugezogen werden, um Informationen für die Prozessierung hinzuzuziehen

Die Stimme wird vorzugsweise mittels eines Vocoders künstlich erzeugt. Das Stimmprofil wird zuvor mit Hilfe von Trainingsdaten eingestellt. Sofern keine Trainingsdaten vorhanden sind, wird ein der originären Stimme ähnliches Profil verwendet.

Je nachdem, wie viel Luft oberhalb des unteren Klappenventils pro Zeiteinheit strömt und wie hoch der Luftdruck unterhalb des unteren Klappenventils ist, ermittelt die Steuereinheit der Prothese die gewünschte Sprechlautstärke und steuert die Klappenstellung. Hört der Patient auf zu sprechen, wird die Klappe oder die Klappen des unteren Klappenventils wieder in ihre Ausgangsstellung (maximal geöffnet) gestellt.

Da die Kehlkopfprothese in den Hals eines Patienten eingesetzt wird, sollte das Gewicht der Prothese etwa 100g nicht überschreiten. Dazu werden bei der Fertigung der Prothese gewichtsreduzierende Maßnahmen implementiert. Eine optionale Wabenstruktur sorgt für eine Stabilität und reduziert aufgrund von Hohlräumen auch das Gewicht. Alternativ hilft - je nach Lokation - eine U-Struktur oder eine Röhrenstruktur, um das Gewicht zu reduzieren, während die Stabilität gewährleistet wird. Ferner kann ein leichtes Metall wie Titan, oder glasfaserverstärkter Kunststoff (GFK) oder kohlenstofffaserverstärkter Kunststoff (CFK) als Innenmaterial verwendet werden. Durch Verwendung solcher Strukturen oder Materialien kann das Gewicht reduziert werden, um dem Patienten einen möglichst natürlichen Tragekomfort zu ermöglichen.

Zur Spannungsversorgung der Prothese wird vorzugsweise ein Akkumulator in Form eines Festkörperakkumulators verwendet. Ein solcher Akkumulator ist eine spezielle Bauweise von Akkumulatoren, wobei nicht nur die Elektroden aus festem Material bestehen, sondern auch das Elektrolyt von einem Festkörper gebildet wird. Da kein Material entweichen kann, haben Festkörperakkumulatoren den Vorteil, dass sie nicht altern können und somit beliebig lange gelagert werden können, dabei beliebig geformt werden können, praktisch und selbst bei großen Temperaturschwankungen keine Leistungseinbußen zeigen. Da als Elektrolytmaterial nicht brennbare Chemikalien verwendet werden können, stellt dies einen großen Sicherheitsvorteil gegenüber herkömmlichen Lithium-Ionen-Akkumulatoren dar.

Mit Hilfe eines Transpondersystems, das in ein Halsband integriert sein kann, kann der Akkumulator der Prothese aufgeladen werden und ggf. zusätzlich Daten übertragen werden. Dabei kann ein Induktionslademechanismus eingesetzt werden, um den Akkumulator aufzuladen.

Das Halsband ist dabei vorzugsweise nicht breiter als die Prothese hoch ist und ist flexibel wie ein Gummiband. Zum Anlegen an den Hals öffnet man es an einem Klettverschluss, legt es um den Hals und verschließt den Klettverschluss wieder. In das Gummiband eingewoben sind spulenförmig angeordnete Drähte, die die Induktionsspule darstellen. In der Prothese ist ebenfalls eine kleine Induktionsspule, die durch das elektromagnetische Feld vom Halsband einen Ladestrom erzeugt, der den Akkumulator der Prothese auflädt.

In der Spule ist vorzugsweise ein Temperatursensor integriert, der durch Abschalten des Ladevorganges zu hohen Temperaturen im Halsgewebe vorbeugt, da sich das Gewebe durch das elektromagnetische Wechselfeld erwärmen kann.

Daten können z.B. über NFC und/oder Bluetooth übertragen werden, alternativ sind proprietäre Übertragungsmedien denkbar. Der Ladezustand des Akkus kann ausgelesen werden. Konfigurationsparameter und Softwareupdates können auf die Prothese übertragen werden. Informationen, das heißt Ladefunktionen und/oder Diagnosemöglichkeiten, Daten und Parameter können für eine weitere Analyse ausgelesen werden. Ein Fehlerlogbuch hilft beispielsweise, die Analyse bei Fehlern zu erleichtern. Außerdem können Verschleiß-Parameter abgerufen werden, beispielsweise zu den Ventilklappen. Die Daten werden vorzugsweise verschlüsselt übertragen.

Um die Gefahr einer nächtlichen Strangulation des Patienten zu vermeiden, wird das Halsband an eine Powerbank angeschlossen, die man Nachts am Körper trägt, z.B. als Gürtelclip, um die Prothese ohne Netzstecker über das Halsband kabellos aufzuladen.

Es ist ebenso ein Halsband mit integrierten Akkumulatoren verwendbar, mittels dessen in der Nacht die Prothese aufgeladen wird, wenn das Halsband angelegt wird. Tagsüber können die Akkumulatoren im Halsband an der Netzsteckdose aufgeladen werden.

Weiterhin ist eine Kombination aus dem mit integrierten Akkumulatoren versehenen Halsband und einer Powerbank, z.B. um die Betriebsdauer der Prothese zu verlängern. Somit sind verschiedene Kombinationen aus Lade- und Transponderfunktionen und/oder integrierten Akkus verwendbar.

Es ist ebenso eine direkte Ladung am Hals eine Alternative, in der der Akku direkt über ein Ladegerät mit einem daran festgelegten Kabel mit einem Magnet-Connector aufgeladen wird. Über einen solchen Magnet-Connector wird das Risiko einer Strangulation in der Nacht verringert.

Eine weitere alternative Lademöglichkeit ist in Form eines Lade-Kopfkissens bereitgestellt. Die Aufladung läuft dann über ein hufeisenförmiges Kissen mit Hilfe von Induktion und lädt den Akku der Prothese.

Die Erwärmung des Körpergewebes und des Bezugs wird beim Ladevorgang berücksichtigt, und als Material kann ein waschbarer Überzug/Bezug wie ein Fleece, Baumwolle, etc.verwendet werden.

Die erfindungsgemäße Kehlkopfprothese macht, soweit möglich, Verwendung von den im Stand der Technik bekannten Bauteilen wie Elektronikbauteilen aus dotiertem Silizium, biologisch verträglichem fungizidem und bakterizidem Material, Fourier-Transformationseinheiten, Vocoderbausteinen ITU-Spezifikation 782 bzw. GSM ETSI-Spezifikation 01.01, speziellen Akkumulatoren anstelle der Lithiumpolymerakkumulatoren, Flash-EEPROM-Bausteinen, sowie gummierter Bleischicht. Darüber hinaus finden Materialien Verwendung wie Goretex, Dacron, High Performance Polymere (Polyamide 12 (VESTAMID), flexible Varianten "VESTAMIT Care ME", transparente Varianten "TROGAMID Care", VESTAKEEP PEEK Polymere, für Langzeitkörperkontakt "Dental" oder "Implant"-Grade VESTAKEEP PEEK Polymere), biokompatible Kunststoffe, in Kombination mit Edelstählen oder Titan, die keinerlei "Auswascheffekt" unterliegen, Silberkunststoff, Polymer Polycaprolacton (PCL), Zinkoxid (ZnO) Nanopartikel, Zinkoxid-Tetrapoden-Nanopartikel (ZOTeN) ZOTeN mit einer höheren Biokompatibilität als ZnO, PCL/ ZOTeN-Kombinationen, geladene Zustände des Zinks in höher konzentrierten ZOTeN-Formulierungen, Materialien mit allergischer Verträglichkeit, waschbarer Überzüge für das Halsband bzw. für das Ladekissen gemäß Sicherheitsanforderungen und -standards der Norm NF EN 60299, Wolle aufgrund hervorragender Wärmeisolation und Aufnahmefähigkeit für Nachtschweiß, Polyesterfasern sowie Fleece aus aufgerauten Polyesterfasern.

Ein künstlicher Kehldeckel kann erfindungsgemäß auf den Patienten angepasst bereitgestellt werden. Dazu kann mit Hilfe eines MRTs oder CTs ein digitales Abbild des Kehldeckels des Patienten erstellt werden. Falls der Patient nicht mehr über einen Kehldeckel verfügt, werden die Maße der Luftröhre ausgemessen. Mit Hilfe einer Software wird ein für den Patienten individueller Kehldeckel als 3D-Zeichnung erstellt. Mit Hilfe eines 3D-Druckes oder eines anderen Produktionsschritts kann dann der individuelle Kehldeckel des Patienten erstellt werden. Das Material des Kehldeckels ist leicht flexibel, so dass Unebenheiten ausgeglichen werden können und die Luftröhre besser verschlossen wird.

Bei der erfindungsgemäßen Kehlkopfprothese drückt allein die Zunge den künstlichen Kehldeckel runter, um die Luftröhre zu verschließen, und eine Ringfeder, die wie der künstlichen Kehldeckel vorzugsweise an der erfindungsgemäßen Kehlkopfprothese festgelegt ist, führt den Deckel in die Ausgangsposition zurück, was dem natürlichen Bewegungsablauf entspricht, da der natürliche Kehldeckel ebenfalls keine eigene Muskulatur besitzt. Wenn im Zusammenhang mit der chirurgischen Maßnahme zur Entfernung des Kehlkopfes auch Teile der Zunge entfernt wurden und ein Herunterdrücken des künstlichen Kehldeckels durch die Zunge nicht mehr möglich ist, kann erfindungsgemäß auch vorgesehen sein, den künstlichen Kehldeckel elektromotorisch oder hydraulisch mittels an dem künstlichen Kehldeckel angeordneter oder darin integrierter Motoren oder hydraulischer Elemente, die von der Steuereinheit angesteuert werden, herabzusenken und so die Luftröhre zu verschließen.

Die kaudalen (dorsalen) Anteile des Kehldeckels sind besonders aus einem gummiähnlichen Material, um eine Abdichtung der Luftröhre gegenüber der Speiseröhre zu gewährleisten. Die kranialen (ventralen) Anteile des Kehldeckels sind vorzugsweise aus einem nicht haftenden, glatten Material, um den Fluss des Speisebreis zu erleichtern.

Da der Kehldeckel in der Prothese grundsätzlich enthalten ist, aber individuell für jeden Kunden angepasst werden kann, lässt sich der künstliche Kehldeckel aus der Prothese entnehmen und durch einen neuen angepassten Kehldeckel ersetzen. Somit ist der neuartige Kehldeckel auch in einer individuell angefertigten Größe möglich.

Nach Einsetzen und initialer Konfiguration muss die Prothese kalibriert werden. Dazu wird mit Hilfe einer Software die gesprochene Stimme des Patienten analysiert. Dies kann in Echtzeit erfolgen. Dabei umfasst die Analyse die stimmhaften und stimmlosen Anteile der Stimme. Diese Anteile werden verglichen mit der Wunschstimme. Falls Daten verfügbar sind, entspricht die Stimme der ursprünglichen Stimme des Patienten, oder, falls keine oder nur wenige geeignete Daten verfügbar sind, einer Stimme, die dieser sehr ähnlich klingt. Mithilfe der Software (d.h., durch Verwendung eines konfigurierten Algorithmus', Synthetisierung der Stimme mithilfe künstlicher Intelligenz, Bereinigung und/oder Vervollständigung des Audiomaterials) wird nun die mit Hilfe der Prothese gesprochene Stimme mit der Wunschstimme verglichen und bei Bedarf optimiert (z.B. Obertöne, Wärme der Stimme, etc.) und ein Stimmmodell angefertigt (AI/KI , um auf Basis alter Audio- und/ oder Videoaufnahmen (sofern vorhanden) ein Stimmmodell zu erstellen, das der Stimme des Patienten ähnlich ist: Audioaufnahmen des Patienten → KI/AI Software → Stimmmodell → Kalibrierung).

Die Konfiguration kann mit Hilfe der Software auch zusätzlich manuell angepasst werden. Die Konfiguration wird dann auf die Prothese übertragen. Der Vorgang kann beliebig oft wiederholt werden, bis die Prothese eine für den Patienten optimale Konfiguration hat. Da die Berechnung und Übertragung der Daten an die Prothese in Echtzeit erfolgen kann, ist das ein sehr schneller und für den Patienten unkomplizierter Prozess.

Die Prothese enthält viele Komponenten, die infolge von Verschleiß ausgetauscht werden müssen. Folgende Komponenten sind erfindungsgemäß vorzugsweise austauschbar, um eine Wartung zu ermöglichen:
- Akku: die Akkukapazität lässt nach einigen Jahren in der Regel nach (ca. alle 2-4 Jahre). Der Akku muss öfter am Tag aufgeladen werden anstatt nur einmal pro Nacht.
- künstlicher Kehldeckel: das Material wird durch Säuren angegriffen, so dass ein vollständiger Verschluss der Luftröhre nicht gewährleistet werden kann. Der künstliche Kehldeckel kann aus der Prothese entnommen und ausgetauscht werden.
- Ventilklappen: das Öffnen und Schließen kann die Ränder verschleißen und damit ein luftdichtes Verschließen verhindern. Über- und Unterdruck können nicht mehr richtig aufgebaut werden. Verschleiß am Lager und Servos.
- Alle elektronischen Komponenten, die durch statische Ströme im Körper, durch Strahlung oder durch Alterung defekt werden.
- Lautsprecher.
- alle (Signal-)Prozessoren und Speicher.

Zusätzlich kann erfindungsgemäß für den Patienten eine Diagnose-Einheit vorgesehen sein, die in das Ladegerät (Powerbank) integriert sein kann.

Die Diagnose-Einheit hat ein Display (z.B. TFT, OLED), auf dem ein Status-Bildschirm zusammenfassend den Zustand der Prothese anzeigt, z.B. grünes O bedeutet, dass alles in Ordnung ist. Erscheint ein gelbes oder gar rotes O, deutet dies auf ein Problem oder einen schweren Fehler hin. Unter dem Display gibt es Soft-Keys, mit denen man durch die Meldungen blättern kann (dies geht auch bei grünem Status, also alles OK). Hier wird der Hinweis gegeben, ob der Patient bald oder sofort den HNO-Arzt aufsuchen soll. Ernst zu nehmende oder sogar lebensbedrohliche Fehlermeldungen werden über ein Frühwarnsystem an den HNO-Arzt oder den Hersteller gemeldet.

Komponenten der Prothese, die überwacht werden bzw. Fehler verursachen können: Mögliche Fehlermeldung:
Eingänge:
   Nervenschnittstellen, Sensoren für Luftdruck und Luftströmung Eingänge: Kein Signal mehr von einem Nerven, Signal zu schwach, Sensorsignal zu hoch, zu niedrig, fehlt (z.B. Luftdruck zu hoch, Luftströmung zu hoch), Sensorwiderstand fehlerhaft
Power:
   Akku-Kontrolleinheit, Akku, Energiemanagement Power: Akku: Anzahl Ladezyklen, noch mögliche Ladungen, Akkuspannung, Akkutemperatur (beim Laden), Ladestrom (Halsband optimal angelegt?), Akku entlädt zu schnell (Verbrauch zu hoch)
SoC, DSP:
   Prozessor, DSPs, D/A-Wandler SoC, DSP:
   Watchdog Timer (Komponenten abgestürzt? Neu gestartet?), DSP fehlerhaft, D/A-Wandler fehlerhaft, System zu heiß, System zu feucht oder feucht geworden Ausgabe:
   Verstärker, Lautsprecher Ausgabe:
   Verstärker defekt, Impedanz des Lautsprechers fehlerhaft

Jede rote Fehlermeldung muss dazu führen, dass schnellstmöglich der HNO-Arzt aufgesucht wird, andernfalls droht der sprichwörtliche Verlust der Stimme. Es gibt nichts, was der Patient selbst in Ordnung bringen könnte, außer vielleicht das Halsband beim Laden etwas besser zu positionieren.

Die erfindungsgemäße Kehlkopfprothese kann auch zweistückig als zweiteilige Prothese ausgeführt sein. Dabei verbleibt ein stationäres Modul (Stationärmodul) im Patienten, und ein entnehmbares Modul (Mobilmodul) kann dem Patienten entnommen werden. Nach der Entnahme des Mobilmoduls sind MRT-, CT- und Röntgenaufnahmen des Patienten möglich, ohne dass die im Mobilmodul vorhandene Hardware durch Strahlung beschädigt werden kann. Während das stationäre Modul individuell für den Patienten angefertigt wird (Breite, Höhe, Tiefe, je nach Resektionsfeldern in der Tumor-OP), muss das entnehmbare mobile Modul immer die gleiche Größe besitzen, um im Falle eines Defekts ohne weitere Anforderungen schnell ausgetauscht werden zu können. Die maximale Größe des austauschbaren Moduls der Prothese orientiert sich somit an dem kleinstmöglichen Stationärmodul abzüglich der Wandstärke des Stationärmoduls. Der künstliche Kehlkopfdeckel ist individuell angepasst und Bestandteil des Mobilmoduls. Bei einem Austausch des Mobilmoduls, das für alle Patienten grundsätzlich einheitlich ist und in Intervallen von 2 bis 3 Jahren ausgetauscht werden kann, kann der künstliche Kehlkopfdeckel aus dem Mobilmodul entfernt und ausgetauscht werden.

In der zweistückigen Ausführungsform findet somit ein Stationärmodul Verwendung, das zum einen die Verbindung zwischen Luftröhre (Trachea) und Rachen (Pharynx) nach der Entnahme des Kehlkopfes wiederherstellt. Des Weiteren ermöglicht das Stationärmodul elektrisch leitfähige Verbindungen zwischen den Nervenschnittstellen und den elektronischen Bauteilen des Mobilmoduls und stellt einen Halterungsmechanismus bereit, der zur gesicherten Aufnahme eines Mobilmoduls, in der Regel durch Einschieben des Mobilmoduls in das Stationärmodul von der Mundhöhle des Patienten aus, ausgelegt ist. Bei Einsetzen des Mobilmoduls in das Stationärmodul werden elektrische Kontakte zwischen den Modulen hergestellt und die Nervenschnittstellen sind über leitfähige Verbindungen wie Kabel über das Stationärmodul mit der Steuereinheit im Mobilmodul verbunden. Vorzugsweise sind die Nervenschnittstellen mit einer kurzen Kabelverbindung zu und auf Höhe der jeweiligen Nerven (Nn. Intercostales, N. laryngeus recurrens, etcpp.) im Stationärmodul angeordnet. Das Stationärmodul ist vorzugsweise aus einem Kunststoff, der innen fungizid und bakterizid ist und außen gut mit der Trachea bzw. dem Pharynx verwachsen kann, gefertigt.

Das Mobilmodul ist in der Regel aus dem gleichen Material wie das Stationärmodul gefertigt und ist in einer einfacheren Form so ausgelegt, dass es die Bauchpresse und Husten ermöglicht und dabei einem Verschlucken vorbeugt, allerdings keine Reproduktion der Stimme des Patienten ermöglicht. Dazu ist es wie im Hauptanspruch angegeben ausgelegt und mit den Klappen oben und unten, Kehldeckel und Klappensteuerung mit allen Subsystemen (Steuereinheit, Akku, Firmware, Nervenschnittstellen) versehen.

In einer Weiterbildung ist das Mobilmodul so ausgelegt, dass es die Reproduktion der Stimme des Patienten ermöglicht, und verfügt dafür zusätzlich über ein Stimmreproduktionssystem mit Lautsprecher und Trichter, die über die Steuereinheit angesteuert werden.

Für die Zeit der Untersuchung und Behandlung des Patienten kann anstelle eines funktionsfähigen Mobilmoduls wie zuvor beschrieben ein vereinfachtes Mobilmodul dem Patienten eingesetzt werden, das lediglich über das obere Klappensystem und Kehldeckel verfügt und keine elektronischen Bauteile umfasst, die bei Bestrahlung Schaden nehmen können. So wird dem Patienten während der Behandlung zumindest das Husten ermöglicht und ein Verschlucken verhindert.

Darüber hinaus kann erfindungsgemäß auch eine Service-Einheit für HNO-Ärzte Verwendung finden. Bei einer solchen Service-Einheit handelt es sich um ein Modul, das die Anbindung des aus dem Patienten entnommenen Mobilmoduls an einen PC zu Diagnose- und Datensicherungszwecken erlaubt. Hierzu besitzt die Ausleseeinheit der Service-Einheit vorzugsweise den gleichen Verschlussmechanismus wie die im Patienten fest implantierte Prothesenhalterung. Über spezielle Service-Kontakte an der Prothesen-Einheit wird die Verbindung zwischen Mobilmodul und PC über die Service-Einheit aufgebaut.

Die Service-Einheit besitzt eine eigene Spannungsversorgung sowie eine Steuerungselektronik, die aus Spannungswandlern, -stabilisatoren, Pegelwandlern und Optokopplern besteht. Die Verbindung mit der USB-Schnittstelle zum PC ist über Optokoppler galvanisch getrennt. Auf dem PC installiert ist die Wartungs-Software, die die Verbindung zur Prothese aufbaut und steuert, Daten aus der Prothese ausliest und schreibt sowie Verbindungen zur zentralen Datenbank des Herstellers aufbaut, um Informationen über die Prothese zu erhalten (Seriennummer, Herstellungsdatum, Daten zum Prothesenträger wie Name, Geburtsdatum, Stimmdateien).

Sollte sich herausstellen, dass das Mobilmodul der Prothese nicht auslesbar ist (z.B. Schäden an der Elektronik oder Akku), so besteht die Möglichkeit, die Daten des "alten" Mobilmoduls (Stimmcharakteristika des Patienten) über die Anbindung an die Datenbank, auch über das Internet, herunterzuladen und auf ein neues Mobilmodul die Austauschprothese zu beschreiben. Das setzt voraus, dass die Stimmcharakteristika aller Patienten in einer zentralen Datenbank gespeichert vorliegen. Diese zentrale Datenbank beinhaltet alle Daten, die für eine Parametrisierung der Prothese notwendig sind, u.a. Filterparameter für die stimmhaften Anteile der Stimme, Trainingsinformationen vor Laryngektomie), Lautstärke-Parameter, Wartungsinformationen (s.o.), etc.

Für den Fall, dass das defekte Mobilmodul auslesbar ist, können die Daten ausgelesen, zwischengespeichert und in ein neues Mobilmodul geladen werden. Das neue Mobilmodul kann anschließend in das Stationärmodul des Patienten eingesetzt werden.

Die Erfindung wird anhand der beigefügten Zeichnungen weiter erläutert. Dabei zeigen:
Figur 1 einen Längsschnitt etwa in der Mitte der erfindungsgemäßen Prothese;
Figur 2 einen Längsschnitt entlang der in Figur 1 mit B B' gezeichneten Ebene;
Figur 3 einen senkrecht zu der durch die Ebenen A' A und B' B aufgestellten Ebenen entlang der in den Figuren 1 und 2 dargestellten Ebene C C';
Figur 4 eine schematische Darstellung der in Figur 1 gezeigten Prothese eingesetzt im Halsbereich eines Patienten;
Figur 5 eine schematische Darstellung der im Halsbereich unterhalb der Zunge eines Patienten eingesetzten Prothese mit um den Hals des Patienten angeordneten Ladeband mit Ladeanschluss und Kontaktstecker;
Figur 6 eine schematische Darstellung eines Patientenmoduls zur Anzeige der Ladestände des Akkus der Prothese und des Akkus der Ladeeinheit.

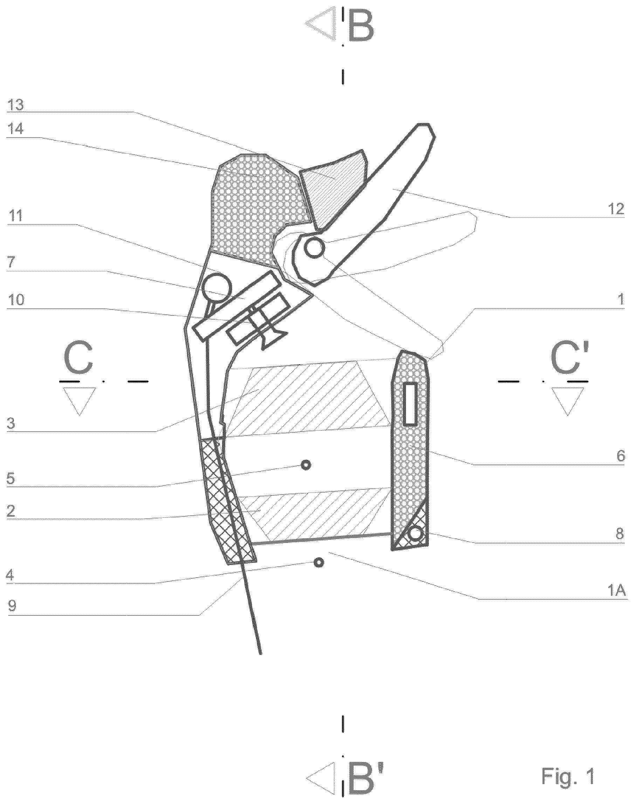

Wie in Figur 1 gezeigt, weist der Prothesenhohlkörper (1) eine den Prothesenkörper durchdringende Ausnehmung (1A) auf. Der Prothesenhohlkörper kann dabei eine hohlzylindrische oder hohlquaderförmige Form besitzen. An dem unteren Ende des Prothesenhohlkörpers, das in der in den Körper des Patienten eingesetzten Position dem oberen Ende der Luftröhre zugeordnet ist, ist mindestens eine Ventilklappe (2) angeordnet, die zwischen einer auf-Stellung und einer geschlossen-Stellung mittels eines motorischen Antriebs, insbesondere elektromotorischen Antriebs, oder hydraulisch verstellbar sein kann. Am oberen Ende des Prothesenhohlkörpers (1), das durch einen künstlichen Kehldeckel (12) verschließbar ist, ist mindestens eine obere Ventilklappe (3) angeordnet, die zwischen einer Auf-Position und einer Geschlossen-Position verstellbar ist. In einer Ausführungsform ist die mindestens eine obere Ventilklappe (3) zwischen zwei entgegengesetzten Auf-Positionen verstellbar, wobei die Geschlossen-Position etwa in der Mitte zwischen den beiden Auf-Positionen vorgesehen ist.

Unterhalb der unteren Ventilklappe (2) ist ein Sensor vorgesehen, mit dem Druck und/oder Geschwindigkeit der vorbeiströmenden Luft gemessen werden kann. Zwischen der mindestens einen unteren Ventilklappe (2) und der mindestens einen oberen Ventilklappe (3) ist optional ein weiterer Sensor (5) vorgesehen, mit dem die Geschwindigkeit und/oder der Druck der im Prothesenhohlkörper durchströmenden Luft gemessen werden kann. Die beiden Sensoren (4) und (5) sind mit der Steuereinheit (7) elektrisch verbunden.

An dem der Luftröhre zugewandten ringförmigen Ende des Prothesenhohlkörpers (1) können Schnittstellen zur Verbindung mit den Nervus laryngeus recurrens (8) und Nervus intercostales (9) vorgesehen sein, die ebenfalls mit der Steuereinheit (7) elektrisch verbunden sind. In den Prothesenhohlkörper (1) ist in der Wand oder am oberen Ende mindestens eine Akkueinheit (6); (14) eingebaut, die zur elektrischen Versorgung der motorischen Antriebe für die Ventilklappen und der Steuereinheit (7) ausgelegt ist.

Die Steuereinheit (7) und der Kehldeckelsenker (13) und Kehldeckel (12) können vorteilhaft an einem Halbhohlkörper (11) als Verlängerung des der Luftröhre abgewandten Endes des Prothesenhohlkörpers (1) angeordnet sein. An dem dem Prothesenhohlkörper (1) zugewandten Bereich des Halbhohlkörpers (11), z.B. eines Halbhohlzylinders, kann vorteilhaft ein Schallwandler (10), beispielsweise in Form eines Lautsprechers, angeordnet sein, der der Spracherzeugung im Prothesenhohlkörpers (1) dient. Am oberen Ende des Halbhohlkörpers (11) kann ebenso ein Akku (14) angeordnet sein, der der Stromversorgung der Steuereinheit (7) und der weiter damit elektrisch verbundenen Bauteile dient.

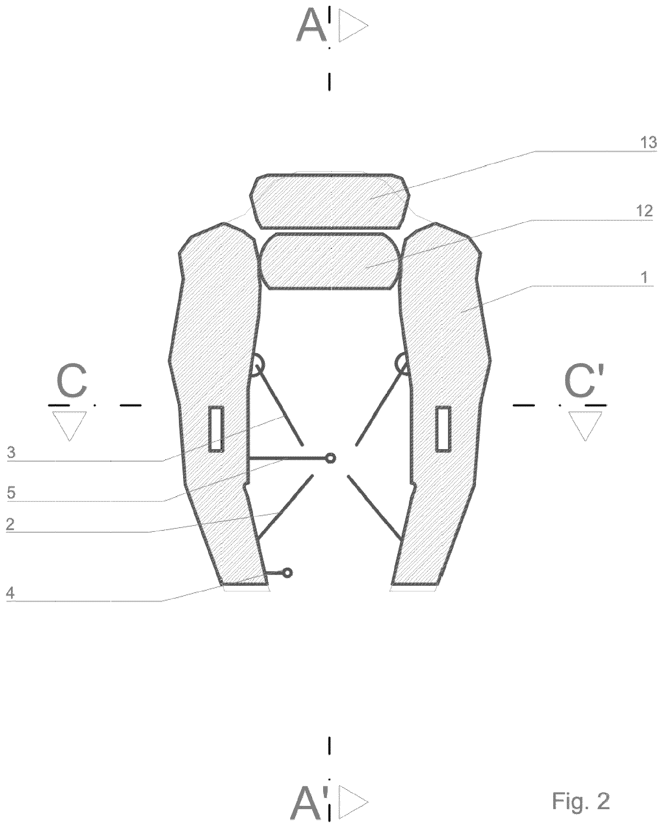

Wie festgehalten, ist in Figur 2 die gegenüber der Darstellung in Figur 1 um 90° entlang der Ebene B B' ein Längsschnitt durch den Prothesenhohlkörper (1) gezeigt.

Die Ventilklappen (2) und (3) in Form von 2 spiegelbildlich in Prothesenhohlkörper (1) angeordneten Ventilklappen (2) und (3) zeigt die Position in der den Durchfluss erlaubenden offenen Position der Ventilklappen. Etwa in der Mitte der Ventilklappen (2) und (3) ist der Sensor (5) angeordnet unterhalb der unteren Ventilklappen (2) der Sensor (4). Im oberen Bereich des Prothesenhohlkörpers ist der künstliche Kehldeckel (12) im Schnitt gezeigt, darüber der Kehldeckelsenker (13).

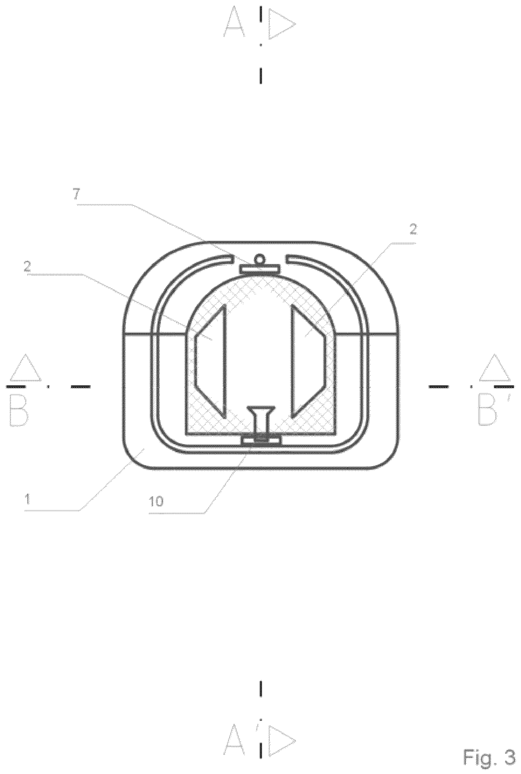

Wie in Figur 3 dargestellt sind die unteren Ventilklappen (2) in der Ausnehmung des Prothesenhohlkörpers (1) in der Draufsicht von oben dargestellt. Beim Schließen der Ventilklappen (2) schließen diese luftdicht mit den Wänden der Ausnehmung in diesem Prothesenhohlkörper (1) ab, und je nach Stellung der Ventilklappen (2) kann in der Ausnehmung ein Luftstrom mit unterschiedlicher Geschwindigkeit eingestellt werden.

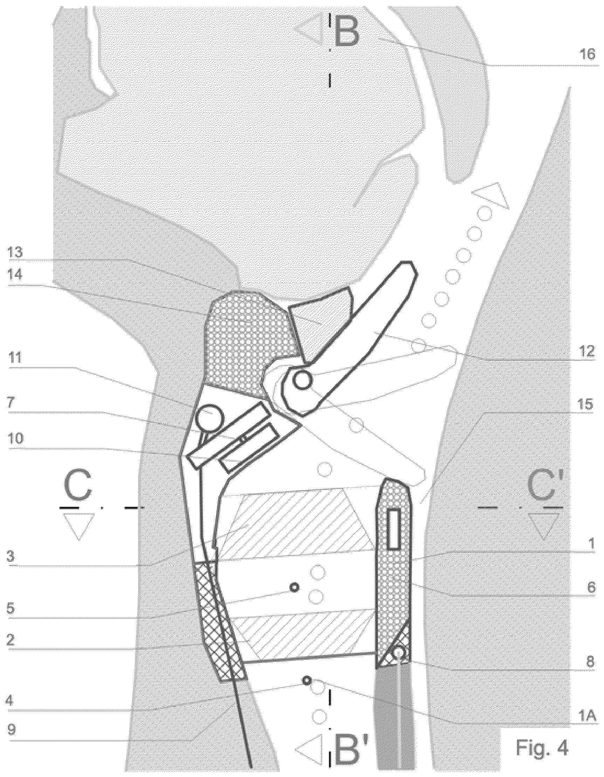

Figur 4 zeigt schematisch die Positionierung der erfindungsgemäßen Kehlkopfprothese mit dem Prothesenhohlkörper (1) im Eingangsbereich der Luftröhre neben der Speiseröhre (15). Unterhalb der Zunge (16) ist der Kehldeckelsenker (13) und der Kehldeckel (12) zum Verschluss des Prothesenhohlkörpers (1) dargestellt. An dem der Luftröhre zugewandten Ende des Prothesenhohlkörpers (1) sind die Nervenschnittstellen (8) und (9) zu den jeweiligen Nerven Nervus laryngeus recurrens (8) und Nervus intercostales (9) dargestellt.

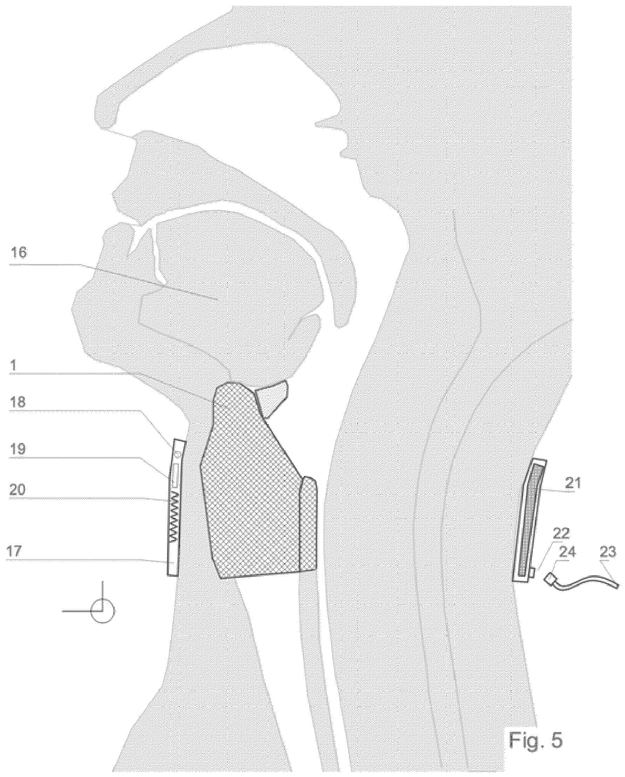

Wie in Figur 5 gezeigt, ist die Kehlkopfprothese im Hals des Patienten vor der Luftröhre angeordnet. Zur Steuerung der Prothese ist, wie in Fig. 1 gezeigt, an dem Prothesenhohlkörper (1) eine Steuereinheit (7) angeordnet, die mit elektromotorischen Einheiten zur Steuerung der Ventilklappen (2) elektrisch leitend verbunden ist. Zur Energieversorgung der Kehlkopfprothese ist in dem Prothesenhohlkörper (1) zumindest ein Akku angeordnet, der durch elektromagnetische Induktion von einem Induktionsladegerät (20), das in einem um den Hals des Patienten gelegten Halsband (17) angeordnet sein kann, in elektromagnetischer Wechselwirkung steht. Zur Steuerung des Ladevorgangs und zur Kommunikation mit der Kehlkopfprothese und deren Steuereinheit kann im Halsband (17) eine Transpondereinheit (18), eine Steuereinheit (19) zur Steuerung des Ladevorgangs und der Kommunikation mit der Kehlkopfprothese, sowie ein Induktionsladegerät (20) vorgesehen sein, das wiederum in leitender Verbindung mit einem Akku (21) nach Art einer Powerbank verbunden ist, dessen Aufladung über einen Ladeanschluss (22) und ein Ladekabel (23) mit Stecker (24) gewährleistet ist.

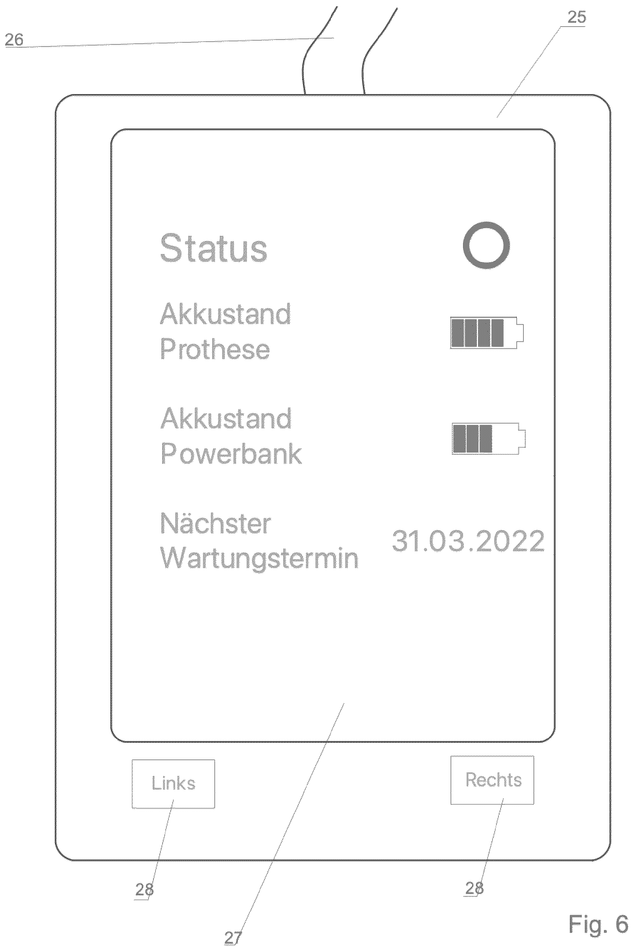

Figur 6 zeigt ein Patientenmodul (25), dass mit der Kehlkopfprothese über Kabel (26) oder eine nicht dargestellte Transpondereinheit mit der Kehlkopfprothese in kommunikativer Verbindung steht. Auf dem Display (27) des Patientenmoduls (25) können Informationen zum Zustand der Kehlkopfprothese, deren Ladezustand oder die Stellung der Ventilklappen angezeigt werden. Die Anordnung von Softkeys (28) kann für verschiedene Displays vorgesehen sein.

### Positionsziffern

- 1:: Prothesenhohlkörper
- 1A:: Ausnehmung
- 2:: Untere Ventilklappen
- 3:: Obere Ventilklappen
- 4:: Luftstromsensor
- 5:: Luftstromsensor
- 6:: Akku
- 7:: Steuereinheit
- 8:: Nervenschnittstelle N. laryngeus recurrens
- 9:: Nervenschnittstelle N. intercostales
- 10:: Schallwandler mit Trichter
- 11:: Halbhohlkörper
- 12:: Künstl. Kehldeckel
- 13:: Kehldeckelsenker
- 14:: Akku
- 15:: Speiseröhre
- 16:: Zunge
- 17:: Halsband
- 18:: Transpondereinheit
- 19:: Steuereinheit
- 20:: Induktionsladegerät
- 21:: Akku
- 22:: Ladeanschluss
- 23:: Ladekabel
- 24:: Ladestecker
- 25:: Patientenmodul
- 26:: Kabel
- 27:: Display
- 28:: Softkey

## Patentansprüche

1. Kehlkopfprothese zur Implantation in die Kavität eines Patienten nach der Entfernung des Kehlkopfes mit einem Prothesenhohlkörper (1) mit einer durchgehenden Ausnehmung (1A):
wobei in der Ausnehmung (1A)
- in dem der Luftröhre in der Einbauposition zugewandten Abschnitt (unterer Abschnitt) der Ausnehmung (1A) zumindest eine Ventilklappe (2) angeordnet ist, deren Öffnungswinkel und/oder der auf die Ventilklappe bei Auslenkung der Ventilklappe aus einer Ausgangsposition wirkenden Rückstellkraft einstellbar ist,
- in dem der Luftröhre in der Einbauposition abgewandten Abschnitt (oberer Abschnitt) der Ausnehmung (1A) zumindest eine Ventilklappe (3) angeordnet ist, die unter Beaufschlagung mit Luftdruck gegen eine Rückstellkraft zwischen zumindest einer offenen Position und einer geschlossenen Position beweglich ist,
- am unteren Ende der Ausnehmung (1A) unterhalb der mindestens einen Ventilklappe (2) ein Luftstromsensor (4) angeordnet ist,
- in der Ausnehmung (1A) zwischen der unteren mindestens einen Ventilklappe (2) und der oberen mindestens einen Ventilklappe (3) optional ein weiterer Luftstromsensor (5) angeordnet ist,
wobei am oberen Ende des Prothesenhohlkörpers (1) ein künstlicher Kehldeckel (12), der zum Verschluss der Ausnehmung (1A) ausgelegt ist und
der zwischen einer offenen Position und einer geschlossenen Position beweglich ist, und eine Feder, die zur Öffnung des Kehldeckels (12) ausgelegt ist, angeordnet sind; und
wobei der Prothesenhohlkörper (1) eine Steuereinheit (7) zur Einstellung der Positionen und der Rückstellkraft der Ventilklappen (2; 3) und zur elektrischen Ansteuerung der Bauelemente der Kehlkopfprothese, mindestens einen Akkumulator (6;14) zur Stromversorgung, eine Transpondereinheit zur Datenübermittlung und zur Aufladung des Akkumulators (6;14) sowie Schnittstellen (8;9) zur Verbindung mit Nerven des Muskel- und Sprechapparates aufweist.

2. Kehlkopfprothese nach Anspruch 1, wobei in dem der Luftröhre in der Einbauposition abgewandten Abschnitt (oberer Abschnitt) der Ausnehmung (1A) mindestens zwei Ventilklappen (3) angeordnet sind, die unter Beaufschlagung mit Luftdruck gegen eine Rückstellkraft zwischen einer offenen Position und einer geschlossenen Position beweglich sind.

3. Kehlkopfprothese nach Anspruch 1 oder 2, wobei in dem der Luftröhre in der Einbauposition zugewandten Abschnitt (unterer Abschnitt) der Ausnehmung (1A) mindestens zwei Ventilklappen (3) angeordnet sind, deren Öffnungswinkel und/oder der auf jede Ventilklappe bei Auslenkung der Ventilklappe aus einer Ausgangsposition wirkenden Rückstellkraft einstellbar sind.

4. Kehlkopfprothese nach Anspruch 1, 2 oder 3, wobei in dem der Luftröhre in der Einbauposition abgewandten Abschnitt (oberer Abschnitt) der Ausnehmung (1A) mindestens zwei Ventilklappen (3) angeordnet sind, die unter Beaufschlagung mit Luftdruck gegen eine Rückstellkraft zwischen einer zueinander offenen Position über eine zueinander geschlossene Position zu einer zweiten zueinander offenen Position beweglich sind.

5. Kehlkopfprothese nach Anspruch 4, wobei die mindestens zwei Ventilklappen (4) jeweils mit einer unterschiedlich einstellbaren Rückstellkraft ausgelegt sind.

6. Kehlkopfprothese nach einem der vorhergehenden Ansprüche, wobei der Prothesenhohlkörper (1) zusätzlich einen Schallwandler (10) nach Art eines Lautsprechers, der über die eine Steuereinheit angesteuert wird, aufweist.

7. Kit mit einer Kehlkopfprothese nach einem der Ansprüche 1 bis 6 und mit einer Vorrichtung zur Aufladung des Akkumulators (6; 14).

8. Kit mit einer Kehlkopfprothese nach einem der Ansprüche 1 bis 6 und mit einer Vorrichtung zur Kommunikation mit der Steuereinheit (7).

9. Kit nach Anspruch 7 oder 8, das eine Kehlkopfprothese nach einem der Ansprüche 1 bis 6 und eine Vorrichtung zur Aufladung des Akkumulators (6;14) und/oder eine Vorrichtung zur Kommunikation mit der Steuereinheit umfasst, wobei die Vorrichtungen jeweils vorzugsweise in ein Halsband integriert sind.
